# EUROPEAN PATENT SPECIFICATION

(11) **EP 2 916 773 B1**
(45) Date of publication and mention of the grant of the patent: **16.08.2017**
(21) Application number: 13716217.8
(22) Date of filing: 25.03.2013
(51) Int. Cl.: A61F 2/28, A61F 2/30, A61F 2/46

(54) **SYSTEM FOR RESHAPING SKULL**
SYSTEM ZUR SCHÄDELUMFORMUNG
SYSTÈME PERMETTANT LE REMODELAGE DU CRÂNE

(30) Priority: 09.11.2012 US 201261724529 P
(43) Date of publication of application: 16.09.2015
(73) Proprietor: IBB Technologie-Entwicklungsfonds GmbH & Co. KG (TEF), 10719 Berlin (DE); Haberl, Ernst-Johannes, 10719 Berlin (DE); Smartt, Nicholas Patrick, 48159 Münster (DE)
(72) Inventor: HABERL, Ernst-Johannes, 10719 Berlin (DE); SMARTT, Nicholas Patrick, 48159 Münster (DE)
(74) Representative: Tegethoff, Sebastian
(86) International application number: PCT/EP2013/056325
(87) International publication number: WO 2014/072082

(56) References cited:
- WO-A1-2012/016200
- US-A- 4 592 352
- US-A- 6 132 437
- US-A1- 2008 070 212
- US-A1- 2012 010 711
- US-B1- 6 331 180
- US-B1- 7 231 723

## Description

### FIELD OF THE INVENTION

The present invention relates to a system intended to aid in the surgical correction of malformed regions of the skull by reshaping bone pieces, particularly in remodeling a skull.

### BACKGROUND OF THE INVENTION

Craniosynostosis is a birth defect in which one or more of the joints between the bones of an infant's skull close prematurely, before the infant's brain is fully formed. Children suffering from craniosynostosis, cannot grow their skull in its natural shape and thus the head is misshapen. Craniosynostosis can affect one or more of the joints in an infant's skull. In some cases, craniosynostosis is associated with an underlying brain abnormality that prevents the brain from growing properly.

Treating craniosynostosis usually means that the infant needs surgery to separate and reshape the fused bones. If there is no underlying brain abnormality, the surgery allows the brain adequate space to grow and develop.

A common problem related to a surgery of an infant suffering from craniosynostosis is to remodel the affected parts of the skull properly. Some important and time-consuming aspects of the surgical operation have so far not been addressed.

One aspect is that the major goal of such an operation is to achieve the most perfect approximation possible to the fictitious "healthy" shape of the patient's skull. Remodeling the 'optimal' shape is completely disposed to the operating surgeon's sense of shape or his or her arbitrary aesthetic criteria, which might not fit with the fictitious natural shape of the patient's skull. Another aspect is that the duration of the operation may be prolonged by corrections, which are required again and again in the sense of groping for the optimum shape. An objective check of success is almost precluded with regards to the individual shape of an infant's skull within its first year of life.

Due to the rigidity of the material, the selection of the shape and the realization in the region of calotte sections presenting different degrees of curvature, for instance in the zone of the forehead, involve a high level of difficulty for the skilled operating surgeon, too.

Thus, the surgeon needs to have a system allowing remodeling bone pieces from malformed parts of a skull with regard to its individual shape of a skull or regions of a skull particularly of a child within its first year of life.

US 2008/0070212 A1 discloses a three-dimensional life-size model of a human child's skull, a kit comprising several models of this kind as well as methods for using the model. The teaching of US 2008/0070212 A1 refers to the fixation of bone pieces to the model by screwing bone pieces onto it.

### OBJECT OF THE INVENTION

It is an objective of the present invention to provide means for reshaping parts of a skull to prepare them for re-implantation.

### BRIEF DESCRIPTION OF THE INVENTION

Within the meaning of the present invention, the terms head template, head model, skull template, and skull model shall be understood synonymously.

The features of claims 1 and 19 solve the object of the invention. The present invention provides a system intended to aid in the surgical correction of malformed regions of the skull, wherein the system comprises a holding frame and at least one bowed clamp-frame arranged around a skull template corresponding to the size and desired head form of a patient, the system comprising a supporting stand for fixing the holding frame at its lateral ends in such a manner that the holding frame is freely rotatable and fixable in any angle, wherein the skull template is firmly connected to the holding frame; said holding frame providing means for fixation of the at least one bowed clamp-frame, the system further comorising at least one clamp-holder for taking up a clamp-plunger and at least one fixation pin for fixation of bone pieces by the at least one bowed clamp-frame onto the skull template.

The system is intended for correction of malformed regions of the skull that are caused by craniosynostosis.

The at least one bowed clamp-frame may have a rectangular cross-section, which advantageously prevents twisting of a fixed clamp-holder. It is intended that the at least one bowed clamp-frame has at least one radial recess to provide at least one predetermined clamping position for the at least one clamp-holder. Further the bowed clamp-frame may be constructed in such a manner that the at least one clamp-holder can be placed on both sides of the at least one bowed clamp-frame. Thus, clamping positions are doubled.

To ease the use of the system according to the invention, the at least one clamp-holder, clamp-plunger and a fixation pin may be preassembled as a clamp assembly. For fixation of the at least one clamp-plunger within the clamp-holder, a ratchet is provided.

The at least one clamp-plunger may have a silicone ring at its end towards the skull template to provide higher friction.

It is intended that the at least one clamp-holder can have a spring-loaded hook to be clipped onto the at least one bowed clamp-frame, wherein the hook engages with an undercut below the at least one bowed clamp-frame.

It is intended further that the material of the at least one fixation pin can be made of metal and it is further intended that the at least one fixation pin has a cap on the end opposite of the skull template. The cap engages in the upper end of the clamp-plunger for fixation in its parking position or clamping position.

For the at least clamp-holder and the clamp-plunger it is intended that they may be made of or are coated with plastic or silicone.

The skull template may have silicone stripes on its surface to provide a high friction holding for the attached bone fragments. Further the skull template may have between the silicone stripes recessed regions to ensure that the fixation pin tips do not touch the skull template.

The skull template may be clipped onto a rigid metal plate that can be screwed into the holding frame for a stable fixation.

A system is further provided with at least one bowed clamp-frames being firmly mounted or preinstalled with the holding frame. The at least one bowed clamp-frame may extend laterally around the skull template. It is also intended that at least one bowed clamp-frame may be parallel to a fictitious horizontal.

A kit comprises the features of claim 19. The kit may comprise skull models of different size and shape.

### BRIEF DESCRIPTION OF THE FIGURES

In the following reference is made to the figures, wherein it is obvious for a person ordinary skilled in the art that the invention is not limited to the embodiments described below.
Figure 1 shows a complete system mounted with a skull template.
Figure 2 shows the holding frame.
Figure 3 shows the bowed clamp-holder.
Figure 4 shows a clamp-assembly.
Figure 5 shows details of a clamp-holder.
Figure 6 shows a clamp-plunger arranged in a clamp-holder with fixation pin.
Figure 7 shows detailed views of the fixation pin.
Figure 8 shows a skull template.
Figure 9 shows a skull template arranged in the holding frame.
Figure 10 shows a clamp-assembly mounted onto a bowed clamp-frame in detail
Figure 11 shows a clamp-assembly in detail
Figure 12 shows a cross-section of a clamp-assembly
Figure 13 shows a bowed clamp-frame extending laterally around the skull template

### DETAILLED DESCRIPTION OF THE INVENTION

The present invention provides a system intended to aid in the surgical correction of a skull, particularly of craniosynostosis in infants.

It is intended that the system shall be based around a set of disposable three-dimensional skull templates 80, but it is also within the scope of the invention to use skull templates 80, which are sterilisable and can be re-used. A skull template 80 corresponding to the size and desired head form of the patient is used as a template to remodel the cranial vault.

An individual skull template 80 may be prepared by establishing shape characteristics of a child's skull from the group of features including the horizontal curvature of the forehead, the vertical curvature of the forehead, the width of the forehead, the fronto-nasal angle, the fronto-occipital diameter, the biparietal diameter and the horizontal radius of curvature of the occipital module, selecting one or more of the established features and preparing a three-dimensional model of a child's skull or parts from the skull from the selected features.

In the surgical procedure, the malformed region of the skull is removed from the patient and cut into several pieces, which are perforated and softened to make them malleable. The bone pieces are reshaped, and clamped against the skull template 80 using (hollow) fixation pins or surgical screws (for instance: Schanz screws) shall be passed through the center hole of each clamp, and tightened to pull the underlying bone pieces onto the screw-clamp. With the bone pieces held stably in position by the clamps and screws or pins, the reconstructed cranial vault can be removed from the skull template 80, but also remodeled in every position of the rotatable holding frame. It is also intended to use Kirschner wires for fixation of the bone pieces.

Small adjustments can be made if necessary to achieve a good fit, after which the reshaped bone pieces are joined together from the inside, using for example resorbable plates and ultrasonically melted resorbable pins. The remodeled cranial vault can then be re-implanted into the patient's head and fixed in position with surgical sutures.

A kit comprising a system according to the present invention shall include skull templates 80 of different shapes and sizes, corresponding to different ages of patient and different natural head forms. The surgeon will then be able to choose the model with the best fitting shape. Thus, the provided system will help to minimize the duration of operation.

The system can be used in conjunction with standard commercially available resorbable implantable plates and fixings, together with commercially available surgical tools such as hand tools, drills and an ultrasonic pin activation tool.

Compared to the usual freehand skull reconstruction technique, the new system and a kit comprising the system as a whole or parts of the system should significantly reduce the duration of the surgical procedure, which in turn reduces the risks to the patient. In addition, the aesthetic success of the operation will be less dependent on the skill of the individual surgeon, allowing less experienced surgeons to achieve successful outcomes for their patients.

### DETAILLED DESCRIPTION OF THE FIGURES

The invention will be described by figures. It is obvious for a person skilled in the art, that the invention will not be limited to the described embodiments.

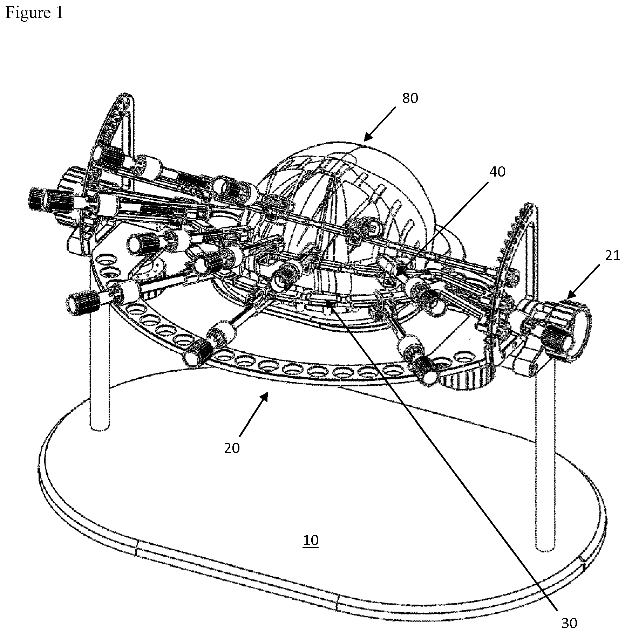

Figure 1 shows the complete system with holding frame 10, bowed clamp-frames 30, clamp-assembly 40 (see below) and skull template 80, mounted onto a supporting stand 10.

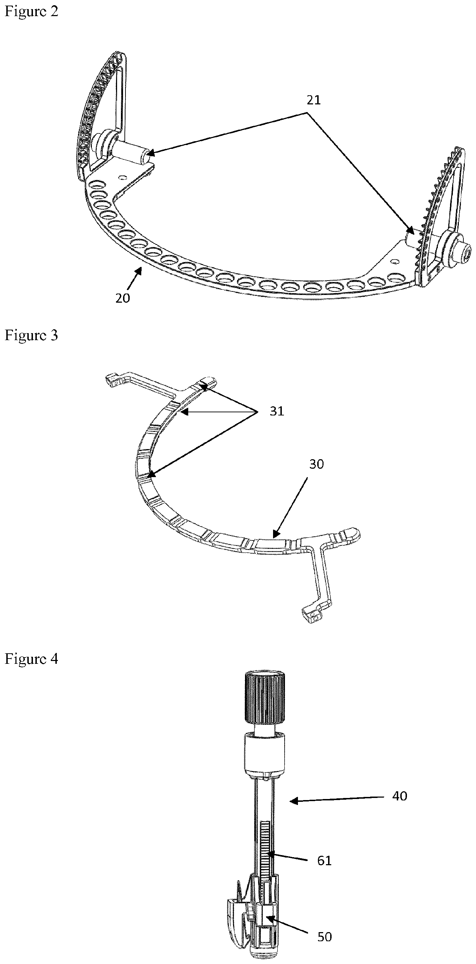

The holding frame 20 (figure 2) provides a rigid structure into which bowed clamp-frames 30 are inserted. The connection 21 between the holding frame 20 (figure 2) and supporting stand 10 shall allow for rotation of the complete holding frame 20, together with the bowed clamp-frames 30, preferably made of high quality steel and reconstructed cranial vault to a desired working angle. It is even possible to invert the fixed and reconstructed bone pieces remodelled onto a skull template 80 to facilitate access to the inside of the cranial vault. The points of rotation of the connection 21 are preferably located laterally of the working area so that no part of the system will hinder the surgeon when working on the inside of the rearranged bone pieces.

It is also intended that at least one or preferably three bowed clamp-frames 30 are firmly mounted or are pre-installed onto the holding frame 20. One of the bowed clamp-frames 30 may be in parallel to a fictitious horizontal, preferably the lowest bowed clamp-frame 30.

The bowed clamp-frames 30 can have a rectangular cross-section, preventing that clamped clamp-holders 50 can twist around a bowed clamp-frame 30. The clamp-frames 30 shall be constructed in such a manner that they have a relatively large vertical height to resist clamping loads, while having a relatively small horizontal dimension to avoid obstructing the surgeon's view during the surgical procedure.

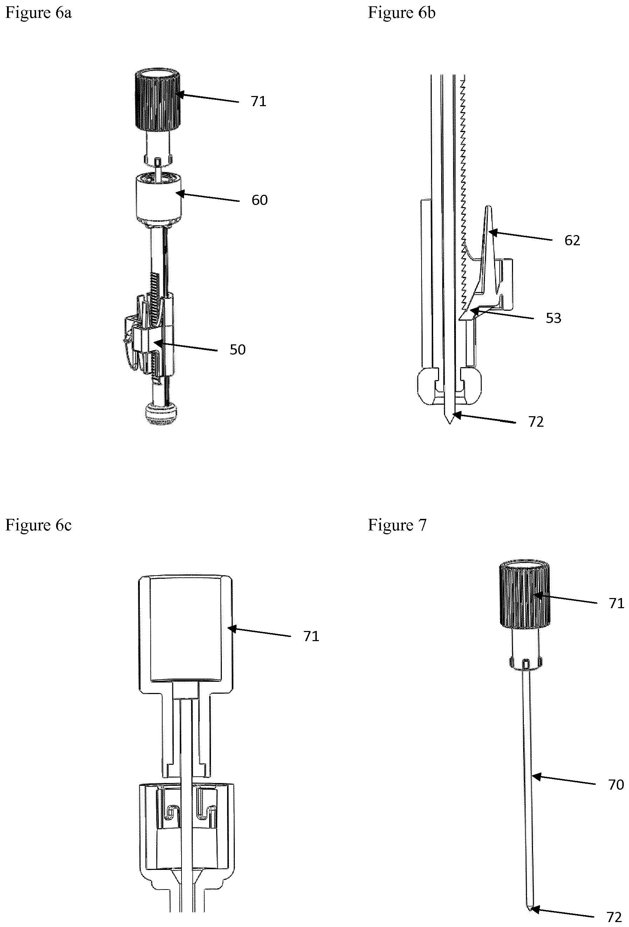

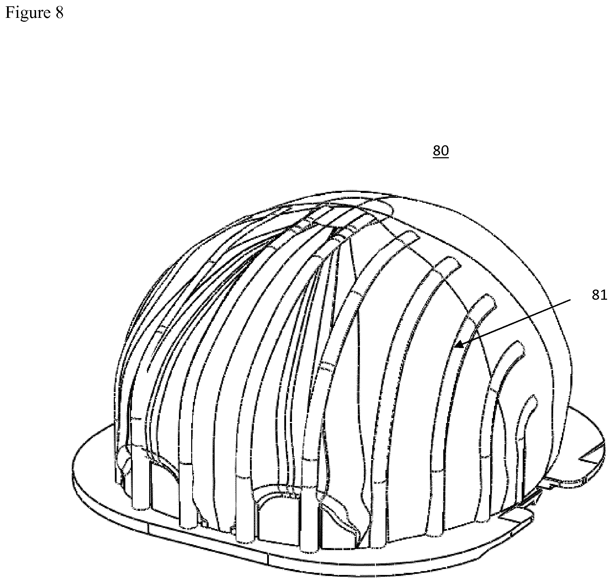

The bowed clamp-frames 30 (figure 3) can include radial recesses 31 to provide a number of distinct clamping positions. The clamp-holder 50 (not shown) shall fit into these recesses 31, ensuring that the bones can only be clamped in defined positions where the tips 72 of the fixation pins 70 (s. figure 7) are not able to contact the skull template 80 (s. figure 8).

The clamp-holder 50 can be placed on either side of the bowed clamp-frames 30, providing the surgeon with a choice of clamping locations. By inverting the clamp-holder 50 180 degrees, each position at a recess 31 provides two different positions for a clamp-holder 50.

The bowed clamp-frames 30, together with clamp-holder 50 and fixation pins 70 are intended to form a pre-assembled disposable sub-assembly. The fixation pins 70 can be preferably made of metal, wherein the cap 71 may be made of plastic. It is further preferred that all other components of the clamp assembly 40 may be made of plastic or silicone. Again, the used material may vary, depending on the particular needs. The term plastic includes particularly synthetic polymers like polyamides.

The clamp assembly 40 (figure 4) can be placed onto and released from the bowed clamp-frame 30 with one hand.

It is an advantage that the clamp-plunger 50 can be pushed against the bone fragments with one hand. It is intended to use a ratchet 61, which prevents them from moving backwards.

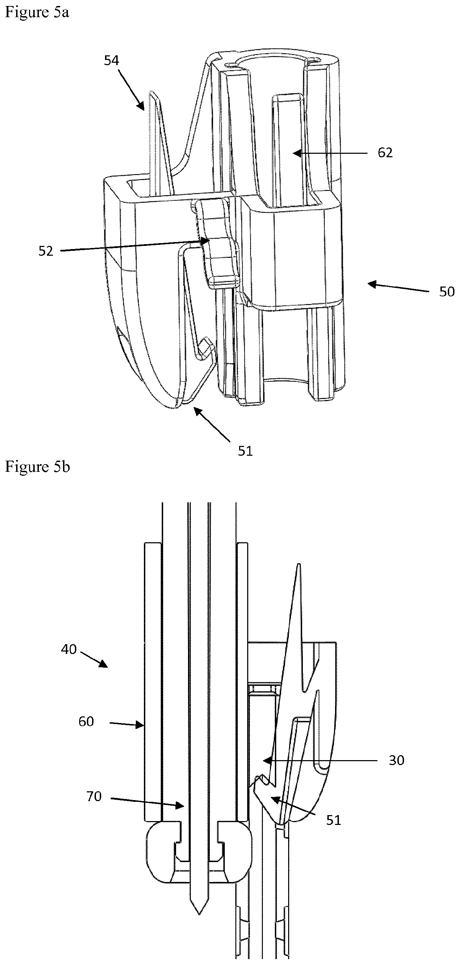

The clamp-holders 50 (figure 5) can be made from injection-molded plastic and can be disposable. The end of the clamp-plunger towards the skull template may comprise silicone or a silicone ring or any other equivalent material to provide a high friction surface.

It is intended that the clamp-holder 50 clip onto the clamp-frame 30 using a (spring-loaded) hook 51, which engages with an undercut below the bowed clamp-frame 30. Spring arms 52 on the side of the clamp-holders 50 biasing the clamp-plunger 60 away from the bowed clamp-frame 30 are intended to ensure the hook 51 remains engaged, also ensuring that audible feedback of correct hook engagement is provided in the form of a click, and tactile feedback in the form of lever movement. A release lever 54 shall be provided, to allow the clamp holder 50 to be released from the bowed clamp-frame 30, for example to reposition the clamp-plunger 50.

The clamp holder 50 including the ratchet pawl 53 (s. figure 6b) and ratchet release lever 62, and the clamp-frame clip with clamp-holder release lever 54, hook 51 and spring arms 52 may be a single injection-molded part as shown in figure 5a.

Figure 5b shows a cross-section of a clamp assembly 40 fixed onto a bowed clamp-frame 30. It has to be noted that the hook 51 is shown in 'as-molded' position, which is overlapping the bowed clamp-frame 30. A fixation pin 70 is inserted into the clamp-plunger 60.

The clamp-plunger 60 may be pre-assembled into a through-hole in the clamp-holder 50 (figure 6). The surgeon can push the clamp-plunger 60 towards the skull template 80, sliding the clamp-plunger 60 over a ratchet 61. The ratchet 61 shall prevent any unwanted outward movement of the clamp-plunger 60 and ensures that the clamping forces can be maintained. A ratchet release lever 62 can be provided to release the ratchet, so that if desired, the ratchet can be released and the clamp-plunger 60 retracted (figure 6b).

In the initial position, the ratchet pawl 53 may be located in a deeper recess. The recess prevents stress relaxation in the ratchet pawl 53 during storage prior to use, ensuring that the ratchet pawl 53 is spring-loaded against the ratchet 61 during normal use.

The intended use of a cap 71 on the end of the bone fixation pins 70 (figure 7) allows the pins 70 to be tightened without the use of a tool and further allows extra torque to be applied and enhances the surgeon feel when screwing into the bone fragments.

A fixation pin 70 shall be pre-assembled into each clamp assembly 40. The pin tip 72 shall be initially retracted, i.e. it is held away from the bone, using a bayonet feature. The pre-assembled fixation pin 70 has the advantage of reducing the time required to assemble the equipment during the operation, and the retracted fixation pin tip 72 reduces the risk of injury to the surgeon during the procedure.

The fixation pin cap 71 may include a bayonet fitting. This prevents the fixation pin 70 from being unintentionally pushed into the bone as the clamp is deployed. To insert the fixation pin 70 into the bone, the surgeon must slightly pull and twist the fixation pin cap 71, before pushing it inwards to a position where it is free to move axially and radially.

After reconstructing the cranial vault, the fixation pins 70 can be pulled back to the initial position, ensuring the sharp fixation pin tips 72 cannot injure the surgeon or patient. Therefore it is intended to use a bayonet fitting, which has to be released in the head of the clamp-plunger 60.

A skull template 80 according to the present invention may comprise the use of silicone stripes 81 to provide high friction holding (figure 8). Bones can be clamped between the silicone stripes 81, so they are less likely to slip. The skull template 80 shall have recessed regions between the silicone stripes 81, ensuring that fixation pin tips 72 (not shown) do not touch the skull template 80.

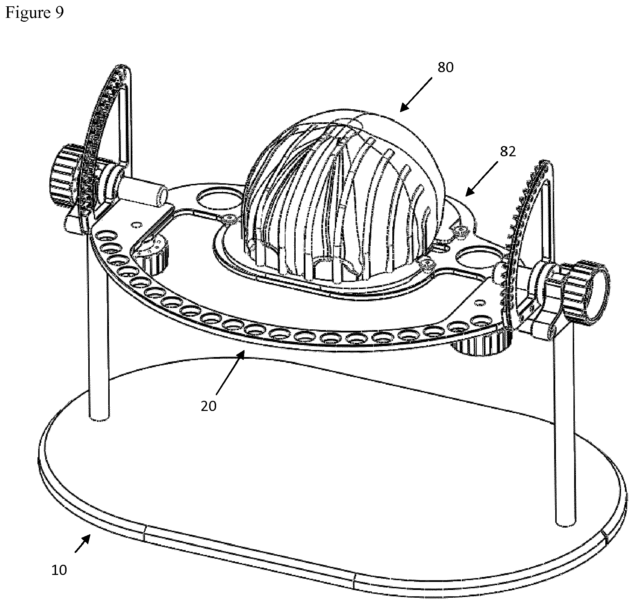

The surgeon can choose between different skull templates 80, which share a common attachment system (clip-in plate) to clip an appropriate skull template 80 onto a rigid metal plate 82 that screws into holding frame 20 (figure 9).

After arranging the bone pieces onto the skull template 80, the reconstructed skull or part of the skull can be inverted before removing the head-form, thereby reducing the risk of bone fragments dropping if the bones are not held securely on the fixation pins 70.

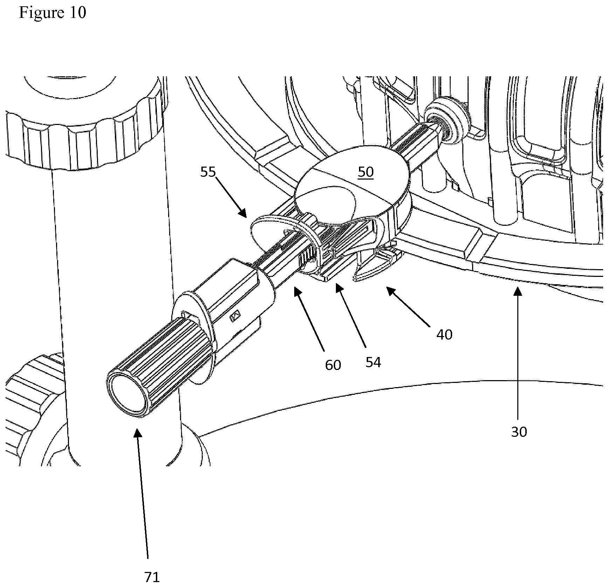

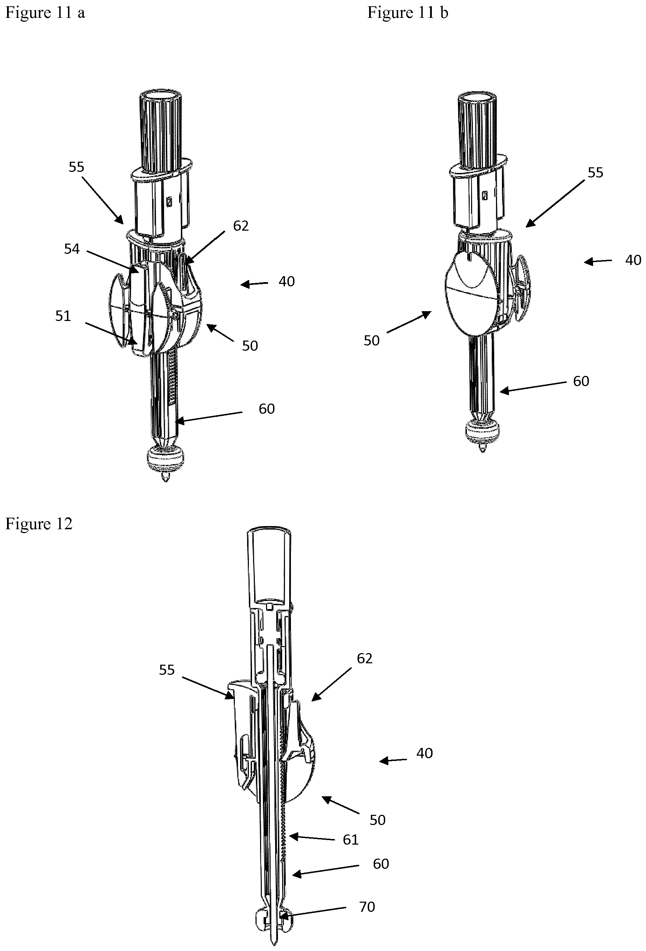

Figure 10 shows another embodiment of a clamp-assembly 40 mounted on a bowed clamp-frame 30. In figure 11a and b both sides of the clamp-assembly 40 are depicted in more detail. One side, the clamp-holder 50 provides a hook 51 for firm assembly with the bowed clamp-frame 30 (not shown). A clamp-holder release lever 54 can release the hook 51, as can be seen in figure 11a.

The clamp-plunger 60 can be adjusted within the clamp-holder 50 by the ratchet release lever 62. In order to provide safe and firm fixation of the chosen position of the clamp-plunger 60 within the clamp-holder 50 a ratchet lock 55 has been added, which can be pushed in order to fix the clamp-plunger 60 within the clamp-holder 50, as can be seen in figures 11a and b.

Figure 12 shows a cross section of the whole clamp-assembly 40, wherein a fixation pin 70 has already been inserted into the clamp-plunger 60. As can be seen on the left side of the clamp-assembly 40 the ratchet lock 55 is pushed down in order to fix the clamp-plunger 60 within the clamp-holder 50. In order to fix the clamp-assembly 40 onto the bowed clamp-frame 30, the clamp-holder 50 will be pushed down onto the bowed clamp-frame 30 until the hook 51 (not shown) of the clamp-holder 50 is securely engaged on the bowed clamp-frame 30 (not shown). In a next step the clamp-plunger 60 has to be pushed until it contacts the bone pieces to be fixed. In order to fix the clamp-plunger 60 in this position the ratchet lock 55 has to be pushed down to lock the clamp-plunger 60 in the clamp-holder 50. In a next step the fixation pin 70 has to be turned at least a quarter clockwise to release it from its "park" position. Next, the fixation pin 70 can be pushed into the bone piece to be fixed and the cap 71 of the fixation pin 70 will be rotated clockwise. In figures 10 to 12 a Kirschner wire is used as a fixation pin 70 to fix bone pieces.

In order to release a fixation pin 70 from a fixed bone piece, the Kirschner wire for instance has to be unscrewed and pulled up into its park position by rotating it a quarter turn anti-clockwise to secure the fixation pin 70 in its park position again. It is an advantage of the invented clamp-assembly 40 that a fixation pin 70 does not need to be completely removed from the clamp-plunger 60.

If the position of the clamp-plunger 60 has to be adjusted, the ratchet lock 55 has simply to be released by pulling it upwards. By pulling the ratchet release lever 62 on the side of the clamp-holder 50 the ratchet 61 can be released. In a next step the clamp-plunger 60 can be adjusted upwards or downwards. The clamp-holder 50 can be released from the bowed clamp-frame 30 (not shown) by pushing the clamp-holder release lever 54 (not shown), if necessary.

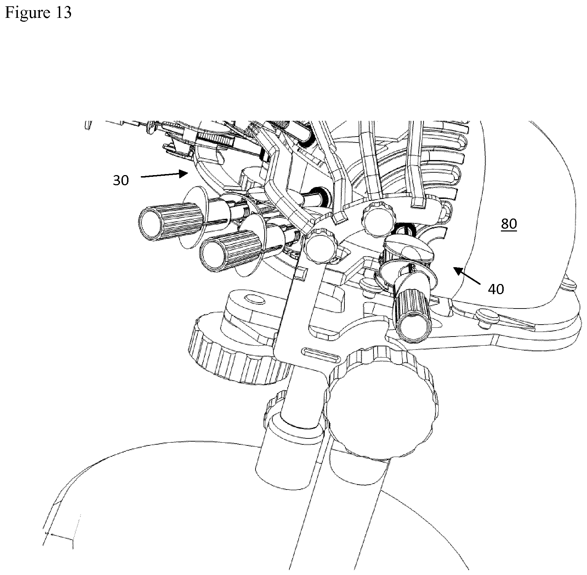

Figure 13 shows a bowed clamp-frame 30 that extends laterally around the skull template 80. A clamp-assembly 40 is depicted that is clipped onto the bowed clamp frame 30 at one side of the skull template 80. Such a bowed clamp-frame extending laterally around the skull template 80 may be preinstalled. It is an advantage that fixation pins 70 (not shown) can be used at a side of the skull template 80.

The system according to the present invention has the following advantages:
- minimized initial set-up time for the equipment
- reduced part count compared to multiple clamps and fasteners
- fast, single handed attachment and removal of clamps to and from the frame
- releasable, ratcheting plunger to clamp bone fragments
- pin tips retracted and held securely during initial clamping of bone fragments
- avoidance of pin tips touching the skull or head template and dislodging fragments of the skull template
- all parts coming into contact with human tissue are disposable (skull template 80; clamps) resulting in reduced cross-contamination risk from bone and brain tissue
- ability to freely set the working angle during the bone setting part of the operation, so that bones can be placed onto a horizontal or near-horizontal working surface, if desired
- high friction surfaces between plunger feet and skull template, so bones do not slide out of place
- quick and easy adjustment of axial position of clamps, allowing loose clamping force to be applied for initial positioning, and tight clamping force for final positioning of bone fragments

The following design alternatives are within the scope of the present invention:
- Separate holding plates on each side of the clamp-frames 30, instead of a rigid holding frame 20.
- Spring rams (detents) to hold bone fixation pin head securely in the bayonet feature; released by twisting against the detent spring force.

### REFERENCE NUMBERS

- 10: Supporting stand
- 20: Holding frame
- 21: Connection
- 30: Bowed clamp-frame
- 31: Radial recess
- 40: Clamp-assembly
- 50: Clamp-holder
- 51: hook
- 52: Spring arms
- 53: Ratchet pawl
- 54: Clamp-holder release lever
- 55: Ratchet Lock
- 60: Clamp-plunger
- 61: Ratchet
- 62: Ratchet release lever
- 70: Fixation pin
- 71: Cap
- 72: Fixation pin tip
- 80: Skull template
- 81: Silicone stripe
- 82: Metal plate

## Claims

1. A system intended to aid in the surgical correction of malformed regions of the skull, wherein the system comprises a skull template (80) corresponding to the size and desired head form of a patient, **characterised in that** the system further comprises a holding frame (20) and at least one bowed clamp-frame (30) arranged around said skull template (80), the system further comprising a supporting stand (10) for fixing the holding frame (20) at its lateral ends in such a manner that the holding frame (20) is freely rotatable and fixable in any angle, wherein the skull template (80) is firmly connected to the holding frame (20); said holding frame (20) providing means for fixation of the at least one bowed clamp-frame (30), the system further comprising at least one clamp-holder (50) for taking up a clamp-plunger (60) and at least one fixation pin (70) for fixation of bone pieces that can thereby be fixed by the at least one bowed clamp-frame (30) onto the skull template (80).

2. The system according to claim 1, wherein the malformed regions of the skull to be corrected are caused by craniosynostosis.

3. The system according to claim 1 or 2, wherein the at least one bowed clamp-frame (30) has a rectangular cross-section.

4. The system according to any one of claims 1 to 3, wherein the at least one bowed clamp-frame (30) has at least one radial recess (31) to provide at least one predetermined clamping position for the at least one clamp-holder (50).

5. The system according to any one of claims 1 to 4, wherein the at least one bowed clamp-frame (30) is constructed in such a manner that the at least one clamp-holder (50) can be placed on both sides of the at least one bowed clamp-frame (30).

6. The system according to any one of claims 1 to 5, wherein the at least clamp-holder (30), clamp-plunger (60) and a fixation pin (70) are preassembled as a clamp assembly (40).

7. The system according to any of claims 1 to 6, wherein ratchets (61) in the at least one clamp-holder (50) enable a fixation of the at least one clamp-plunger (60) in the clamp-holder (50).

8. The system according to any of claims 1 to 7, wherein the at least one clamp-plunger (60) has a silicone ring at its end directed towards the skull template (80).

9. The system according to any of claims 1 to 8, wherein the at least one clamp-holder (50) has a spring-loaded hook to be clipped onto the at least one bowed clamp-frame (30) engaging with an undercut below the at least one bowed clamp-frame (30).

10. The system according to any of claims 1 to 9, wherein the at least one fixation pin (70) is made of metal.

11. The system according to any of claims 1 to 10, wherein the at least one fixation pin has a cap on the end opposite of the skull template.

12. The system according to any of claims 1 to 11, wherein the at least clamp-holder and the clamp-plunger are made of or coated with plastic or silicone.

13. The system according to any of claims 1 to 12, wherein the skull template has silicone stripes on its surface to provide a high friction holding for the attached bone fragments.

14. The system according to any of claims 1 to 13, wherein the skull template has recessed regions between the silicone stripes to ensure that the fixation pin tips do not touch the skull template.

15. The system according to any of claims 1 to 14, wherein the skull template is clipped onto a rigid metal plate that screw into the holding frame (20).

16. The system according to any of claims 1 to 15, wherein at least one bowed clamp-frame is firmly mounted or preinstalled to the holding frame (20).

17. The system according to any of claims 1 to 16, wherein at least one bowed clamp-frame extends laterally around the skull template.

18. The system according to any of claims 1 to 17, wherein at least one bowed clamp-frame is parallel to a fictitious horizontal.

19. A kit comprising a system according to any of claims 1 to 18, the kit comprising a metal plate (82) for mounting the skull template, the at least one bowed clamp-frame (30) is adapted for fixing at it at least one clamp-assembly (40) comprising at least a clamp-holder (50), clamp-plunger (60) and at least one fixation pin (70).

20. A kit according to claim 19 comprising skull templates of different size and shape.

## Patentansprüche

1. Ein System zur Unterstützung der chirurgischen Korrektur von missgebildeten Schädelregionen, wobei das System eine Schädelvorlage (80) enthält, welche der Größe und gewünschten Kopfform eines Patienten entspricht, **dadurch gekennzeichnet, dass** das System weiterhin einen Halterrahmen (20) und mindestens einen um besagte Schädelvorlage (80) herum angeordneten gebogenen Klemmrahmen (30) enthält, das System weiterhin enthaltend einen Ständer (10) zur Befestigung des Halterahmens (20) an seinen lateralen Enden derart, dass der Halterrahmen (20) frei drehbar und in jedem Winkel fixierbar ist, wobei die Schädelvorlage (80) fest mit dem Halterrahmen (20) verbunden ist; wobei der Halterrahmen (20) Mittel zur Befestigung des mindestens einen gebogenen Klemmrahmens (30) aufweist, und das System weiterhin mindestens einen Klemmhalter (50) zur Aufnahme eines Klemmkolbens (60) und mindestens einen Befestigungsstift (70) zur Fixierung von Knochenstücken umfasst, welche dadurch mittels des mindestens einen gebogenen Klemmrahmen (30) auf der Schädelvorlage (80) fixiert werden können.

2. Das System nach Anspruch 1, wobei die zu korrigierenden missgebildeten Schädelregionen durch Craniosynostose verursacht sind.

3. Das System nach Anspruch 1 oder 2, wobei der mindesten eine gebogene Klemmrahmen (30) einen rechteckigen Querschnitt hat.

4. Das System nach einem der Ansprüche 1 bis 3, wobei der mindesten eine gebogene Klemmrahmen (30) mindestens eine radiale Aussparung (31) hat um mindestens eine vorgegebene Klemmposition für den mindestens einen Klemmhalter (50) zu stellen.

5. Das System nach einem der Ansprüche 1 bis 4, wobei der mindesten eine gebogene Klemmrahmen (30) so konstruiert ist, dass der mindestens eine Klemmhalter (50) auf beiden Seiten des mindestens einen gebogenen Klemmrahmen (30) platziert werden kann.

6. Das System nach einem der Ansprüche 1 bis 5, wobei der mindesten eine gebogene Klemmrahmen (30), Klemmkolben (60) und ein Befestigungsstift (70) als Klemmeinheit (40) vormontiert sind.

7. Das System nach einem der Ansprüche 1 bis 6, wobei Ratschen (61) in dem mindestens einen Klemmhalter (50) eine Fixierung des mindestens einen Klemmkolbens (60) im Klemmhalter (50) ermöglichen.

8. Das System nach einem der Ansprüche 1 bis 7, wobei der mindesten eine Klemmkolben (60) an seinem gegen die Schädelvorlage (80) gerichteten Ende einen Silikonring hat.

9. Das System nach einem der Ansprüche 1 bis 8, wobei der mindestens eine Klemmhalter (50) einen federbelasteten Haken hat, der an dem mindestens einen gebogenen Klemmrahmen (30), der in einen Hinterschnitt unter dem zumindest einen gebogenen Klemmrahmen (30) greift, befestigt werden kann.

10. Das System nach einem der Ansprüche 1 bis 9, wobei der mindestens eine Befestigungsstift (70) aus Metall gemacht ist.

11. Das System nach einem der Ansprüche 1 bis 10, wobei der mindestens eine Befestigungsstift am der Schädelvorlage gegenüber liegenden Ende eine Kappe hat.

12. Das System nach einem der Ansprüche 1 bis 11, wobei der mindestens eine Klemmhalter und der Klemmkolben aus Plastik oder Silikon gemacht oder beschichtet sind.

13. Das System nach einem der Ansprüche 1 bis 12, wobei die die Schädelvorlage Silikonstreifen auf ihrer Oberfläche hat, um für die angebrachten Knochenfragmente eine hohe Reibung zu schaffen.

14. Das System nach einem der Ansprüche 1 bis 13, wobei die Schädelvorlage zwischen den Silikonstreifen ausgesparte Regionen hat um sicherzustellen, dass die Befestigungsstifte die Schädelvorlage nicht berühren.

15. Das System nach einem der Ansprüche 1 bis 14, wobei die Schädelvorlage an einer rigiden Metallplatte befestigt ist, die in den Halterrahmen (20) schraubt.

16. Das System nach einem der Ansprüche 1 bis 15, wobei der mindestens eine gebogene Klemmrahmen fest montiert oder vormontiert ist an den Halterrahmen (20).

17. Das System nach einem der Ansprüche 1 bis 16, wobei der mindestens eine gebogene Klemmrahmen sich lateral um die Schädelvorlage erstreckt.

18. Das System nach einem der Ansprüche 1 bis 17, wobei der mindestens eine gebogene Klemmrahmen parallel ist zu einer fiktiven Horizontalen.

19. Ein Kit enthaltend ein System nach einem der Ansprüche 1 bis 18, das Kit enthaltend eine Metallplatte (82) für die Montage der Schädelvorlage, den mindestens einen gebogenen Klemmrahmen (30) der dazu geeignet ist, an ihm mindestens eine Klemmeinheit (40) zu befestigen, die mindestens einen Klemmhalter (50), einen Klemmkolben (60) und mindestens einen Befestigungsstift (70) enthält.

20. Ein Kit nach Anspruch 19 enthaltend Schädelvorlagen unterschiedlicher Größe und Form.

## Revendications

1. Système destiné à faciliter la correction chirugical de régions mal formées du crâne, dans lequel le système comprend un modèle de crâne (80) correspondant à la taille et à la forme de tête souhaitée d'un patient, charactérisé en ce que le système comprend en outre un cadre de maintien (20) et au moins un cadre de serrage courbé (30) arrangé autour du modèle du dit modèle de crâne (80), le système comprenant en outre un support soutenant (10) pour fixer le cadre de maintien (20) à ses extrémités latérales de telle sorte que le cadre de maintien (20) peut être tourner librement et être fixé dans n'importe quel angle, dans lequel le modèle de crâne (80) est relié fermement au cadre de maintien (20); dans lequel le cadre de maintien (20) présente des moyens de fixation du au moins un cadre de serrage courbé (30), et le système comprends en outre au moins un support de serrage (50) pour prendre un piston de serrage (60) et au moins une tige de fixation (70) pour la fixation de morceaux d'os qui peuvent ainsi être fixés sur le modèle de crâne (80) au moyen du au moins un cadre de serrage courbé (30).

2. Le sysème selon la revendication 1, dans lequel les régions mal formées du crâne à corriger sont provoquées par une craniosynostose.

3. Le sysème selon la revendication 1 ou 2, dans lequel le au moins un cadre de serrage courbé (30) présente une section transversale rectangulaire.

4. Le sysème selon l'une quelconque des revendications 1 à 3, dans lequel le au moins un cadre de serrage courbé (30) présente au moins une cavité radiale (31) pour fournir au moins une position de serrage prédéterminée pour au moins un support de serrage (50).

5. Le sysème selon l'une quelconque des revendications 1 à 4, dans lequel le au moins un cadre de serrage courbé (30) est construit de telle sorte que le au moins un support de serrage (50) peut être placé des deux côtés du au moins un cadre de serrage courbé (30).

6. Le sysème selon l'une quelconque des revendications 1 à 5, dans lequel le au moins un cadre de serrage courbé (30), piston de serrage (60) et tige de fixation (70) sont préassemblées comme assemblage de serrage (40).

7. Le sysème selon l'une quelconque des revendications 1 à 6, dans lequel des cliquets (61) dans le au moins un support de serrage (50) permettent une fixation de l'au moins un piston de serrage (60) dans le support de serrage (50).

8. Le sysème selon l'une quelconque des revendications 1 à 7, dans lequel le au moins un piston de serrage (60) a un anneau en silicone à son extrémité dirigée vers le modèle de crâne (80).

9. Le sysème selon l'une quelconque des revendications 1 à 8, dans lequel le au moins un support de serrage (50) a un crochet à ressort pour être clipsé sur le au moins un cadre de serrage courbé (30) s'engageant avec une contre-dépouille au dessous du au moins un cadre de serrage courbé (30).

10. Le sysème selon l'une quelconque des revendications 1 à 9, dans lequel la au moins une tige de fixation (70) est en métal.

11. Le sysème selon l'une quelconque des revendications 1 à 10, dans lequel la au moins une tige de fixation a un capot à l'extrémité opposée du modèle de crâne.

12. Le sysème selon l'une quelconque des revendications 1 à 11, dans lequel le au moins un support de serrage et le piston de serrage sont fabriqués ou revêtus de plastique ou de silicone.

13. Le sysème selon l'une quelconque des revendications 1 à 12, dans lequel le modèle de crâne possède des bandes des silicone sur sa surface pour fournir une tenue à haute friction aux fragments d'os attachés.

14. Le sysème selon l'une quelconque des revendications 1 à 13, dans lequel le modèle de crâne a des régions en retrait entre les bandes de silicone pour assurer que les tiges de fixation ne touchent pas le modèle de crâne.

15. Le sysème selon l'une quelconque des revendications 1 à 14, dans lequel le modèle de crâne est clipsé sur une plaque métallique rigide qui se visse dans le cadre de maintien (20).

16. Le sysème selon l'une quelconque des revendications 1 à 15, dans lequel le au moins un cadre de serrage courbé est monté de manière fixe ou préinstallé sur le cadre de maintien (20).

17. Le sysème selon l'une quelconque des revendications 1 à 16, dans lequel le au moins un cadre de serrage courbé s'étend latéralement autour du modèle de crâne.

18. Le sysème selon l'une quelconque des revendications 1 à 17, dans lequel le au moins un cadre de serrage courbé est parallèle à une horizontale fictive.

19. Kit comprenant un système selon l'une quelconque des revendications 1 à 18, le kit comprenant une plaque métallique (82) pour monter le modèle de crâne, le au moins un cadre de serrage courbé (30) qui est adapté pour y fixer au moins un assemblage de serrage (40) comprenant au moins un support de serrage (50), un piston de serrage (60) et au moins une tige de fixation (70).

20. Un kit selon la revendication 19 comprenant des modèles de crâne de différentes tailles et formes.
